# EUROPEAN PATENT SPECIFICATION

(11) **EP 3 055 664 B1**
(45) Date of publication and mention of the grant of the patent: **13.03.2019**
(21) Application number: 13783834.8
(22) Date of filing: 23.10.2013
(51) Int. Cl.: G01N 3/303, E02D 1/02, G01N 33/24

(54) **FALLING WEIGHT DEFLECTOMETER**
FALLGEWICHTS-DEFKLEKTOMETER
DÉFLECTOMÈTRE À MASSE TOMBANTE

(30) Priority: 11.10.2013 WO PCT/DK2013/050321
(43) Date of publication of application: 17.08.2016
(73) Proprietor: Dynatest International A/S, 2860 Søborg (DK)
(72) Inventor: MADSEN, Jakob Find, DK-2600 Glostrup (DK)
(74) Representative: AWA Denmark A/S
(86) International application number: PCT/DK2013/050340
(87) International publication number: WO 2015/051798

(56) References cited:
- WO-A1-2012/120179
- CN-U- 202 928 904
- CN-Y- 2 869 109
- DE-U1- 9 305 327
- JP-A- 2008 020 424
- JP-A- 2008 275 518
- US-A- 2 890 766
- US-A- 3 485 083
- US-A- 3 998 090
- US-A- 4 359 890

## Description

The present invention relates to falling weight deflectometers.

A falling weight deflectometer (FWD) is a non-destructive testing device used by civil engineers to evaluate the physical properties of pavement structures. Data from the FWD is primarily used to estimate the load carrying capacity, of *inter alia* pavements comprising Portland cement concrete (PCC) or asphalt concrete (AC) surfaces. Use includes roads, airport pavements, and railway tracks. The falling weight deflectometer is typically integrated in a trailer that can be towed to a test location by another vehicle, but can also be integrated in the vehicle itself.

The falling weight deflectometer is designed to impart a load pulse to the pavement surface which simulates the load produced by a rolling vehicle wheel. The load is produced by dropping a large weight on a set of rubber buffers. The resulting force is transmitted to the pavement through a circular load plate. A load cell or other load sensing transducer is mounted atop the load plate and measures the applied load on the pavement surface. A series of deflection sensors such as geophones are mounted linearly along a beam extending from the centre of the load plate and measure the resulting pavement surface deflection in response to the load at a variety of distances from the centroid of the applied load. The FWD data may be used to calculate stiffness-related parameters, e.g. Young's modulus of the layers of a multilayered pavement structure, such as the pavements of roads or airports comprising PCC or AC.

In prior art FWDs the weight is typically raised hydraulically along a vertical guide rod or column and held at a predetermined height by a catch mechanism until measurement is to be performed and a drop command is issued by the FWD operator. The catch mechanism is then released and the weight drops onto a force transmitting means, which includes a circular load plate of known diameter, which prior to the dropping of the weight has been, lowered to make contact with the pavement surface at the desired measurement location. Between the force transmitting means and the weight a damping means, e.g. buffers in the form of rubber blocks, is provided in order to transmit an ideally half-sinusoidal impact force to the pavement. The force transmitting means normally comprises a force sensing means so as to measure the actual impact force. Since the drop-weight mass is substantial, typically between 50 and 750 kg, the drop-weight is normally picked up and lifted back to the latched position by a hydraulic lifting mechanism or the like. Prior art FWDs as described above are e.g. known from JP-A-2007-205955 and DE-U-9305327. Examples of prior art FWDs are also described in the article "Faldloddets historie", Bohn, Axel O., Asfalt, pages 4-11, September 1989, ISSN 0004-4318, describing the development of the falling weight deflectometer from 1964 to 1989. JP-A-2008-275518 describes a prior art FWD where the weight is lifted manually. Other examples of related prior art devices are shown in documents : JP2008/020424, US3998090, WO2012/120179, JP2008275518, CN202928904 and US4359890.

One major drawback with such prior art FWDs is that a measurement cycle takes relatively long time, in particular if successive measurements are to be made at the same location. A major reason for this is that the process of the hydraulic lifting means being lowered to pick up the weight, raising the weight to the latch, and disengaging weight from the hydraulic lifting means is relatively slow. Moreover, after a few repetitive drops at one location, the load plate must be raised to the transport position and secured, and lowered again at the next measurement position. In the prior art the latter, is a major contributor to the long test cycles. When thousands of consecutive measurements are to be made along a road to be surveyed, accumulated durations of the processes reduce the efficiency of the overall survey.

On this background it is the object of the present invention to provide a falling weight deflectometer, which allows consecutive measurements to be made at shorter intervals and thereby enhance the testing rate and the overall productivity of pavement deflection measurements.

According to a first aspect of the present invention this object is achieved by a falling weight deflectometer comprising a load plate adapted to engage a test surface, a force transmission means adapted to transmit a force to the load plate, a buffer means, a weight adapted to impact said force transmission means via said buffer means so as to provide a force to be transmitted to said load plate via said force transmission means, a guide means for guiding said weight towards said force transmission means, and a lifting means for lifting said weight to a predetermined height above said force transmission means, characterized in that said lifting means comprises an electric motor driving a threaded spindle in engagement with the weight, and in that the threaded spindle is in a non self-locking engagement with the drop weight.

By using a threaded spindle in engagement with the weight, the lifting mechanism is integrated with the falling weight, making it possible to raise the weight back to the top position immediately after it has been dropped to impact. In particular, because the lifting mechanism is integrated with the falling weight, separate action of attaching it to a lifting mechanism is necessary in order to lift it back to the top position. Moreover, the use of the threaded spindle allows the load plate and force transmitting means to be quickly and efficiently moved to a raised transport position.

This is in particular the case when, according to a preferred embodiment, the threaded spindle is in permanent engagement with the weight. In that case, the return motion, when the weight bounces back upon impact due to the buffer means and other elasticity in the overall system, may simply be continued by controlling the motor suitably.

According to another preferred embodiment, the threaded spindle is in permanent engagement with the electric motor. With the threaded spindle in permanent engagement with both the weight and the electric motor no couplings or the like need to engage, and the return motion may simply be directly continued by controlling the motor suitably. Even though this involves the falling weight forcing the electric motor to rotate and work as a generator, experience has shown that this does not impede the fall of the weight, and that a sufficiently forceful impact may still be achieved. Integrating all three parts, i.e. electric motor, threaded spindle, and falling weight, in one kinematic train substantially simplifies the construction of the falling weight deflectometer.

According to a further preferred embodiment, the threaded spindle is directly driven by the electric motor. Modern permanent magnet motors are fully capable of providing sufficient torque directly on the spindle to lift the weight, even if the threaded spindle has a high lead angle. This further simplifies the construction of the falling weight deflectometer.

In this respect, the lead angle is according to a further preferred embodiment so high that the threaded spindle is in a non self-locking engagement with the weight. Having a high lead angle is advantageous as it allows the falling weight to fall almost freely, i.e. with very little resistance, thus providing an adequate impact force. Thus, according to yet another preferred embodiment, the threaded spindle has a lead angle exceeding 40°, preferably exceeding 50°. This allows the weight to fall without being influenced too much by losses caused by rotating the electric motor which at this point acts as a generator. The electric motor has been carefully chosen so as to not generate too much counter electromotive force, i.e. less than 600 V in order not to risk damage of the control electronics.

According to another preferred embodiment, the engagement between the threaded spindle and the weight is selected among leadscrew, roller screw, or ball screw engagement. This provides low friction, and the falling of the weight is thus even less impeded.

According to a further preferred embodiment, falling weight deflectometer further comprises a motor controller for the electric motor, said motor controller being adapted to control the speed of the falling weight. By having a motor controller adapted to control the speed of the falling weight it becomes possible to influence the falling weight in numerous advantageous ways, *inter alia* allowing more controlled impacts as compared to the simple drop of the prior art.

According to yet a further preferred embodiment, falling weight deflectometer further comprises a motor controller for the electric motor, said motor controller being adapted to control the position of the falling weight. By having a motor controller adapted to control the position of the falling weight it becomes possible to influence the falling weight in numerous advantageous ways, *inter alia* allowing more controlled impacts as compared to the simple drop of the prior art.

According to a further preferred embodiment, the falling weight deflectometer comprises a frame and means for securing the drop weight with respect to the frame. Securing the drop weight allows the use of the threaded spindle to lift and lower the force transmission means, more specifically lowering it into engagement with the test surface an lifting it into a transport position by means the electric motor.

The present invention will now be described in greater detail with reference to the drawings on which:
Fig. 1 shows a schematic perspective view of the core parts of a falling weight deflectometer according to the invention,
fig. 2 a schematic perspective view of a different embodiment of the falling weight deflectometer including parts of the frame,
fig. 3 shows a schematic perspective view of the falling weight deflectometer of fig. 1,
fig. 4 shows a schematic diagram of elevation versus time for a drop and lift sequence in the prior art,
fig. 5 shows a schematic diagram of elevation versus time for a drop and lift sequence according to the present invention, and
figs. 6a-6f schematically show the lowering of the FWD load plate onto the surface and a subsequent drop of the weight.

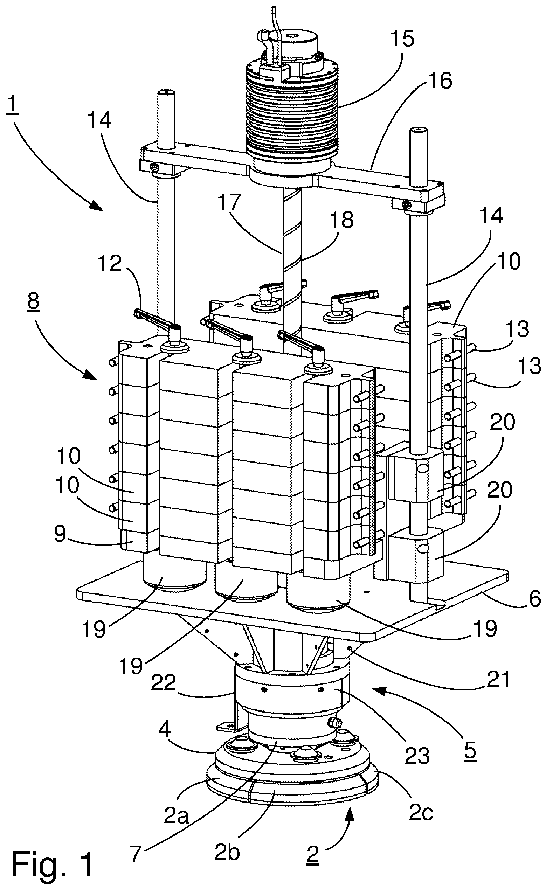

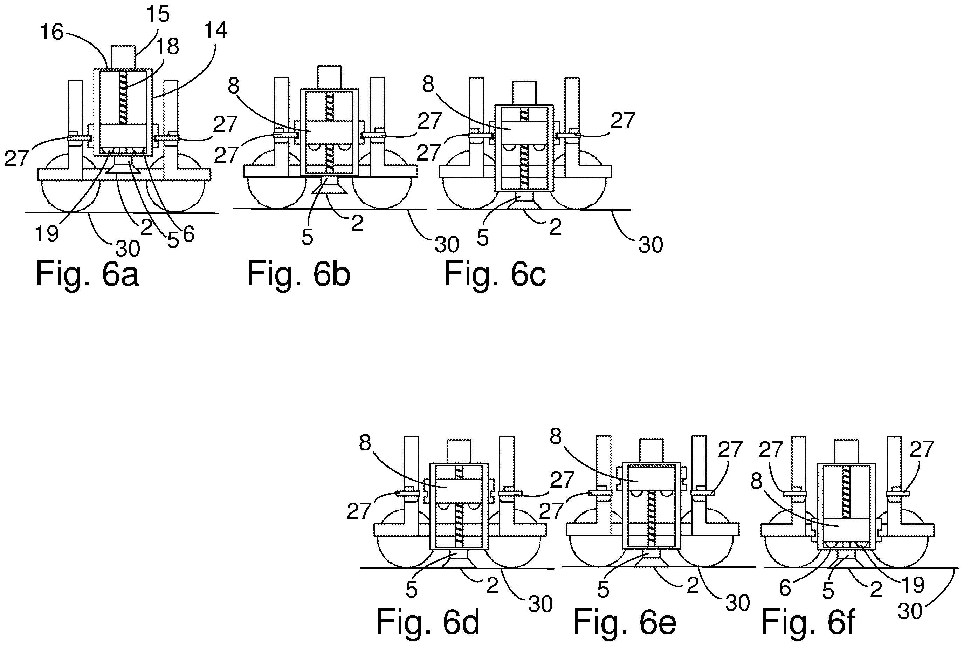

Turning first to fig. 1, a perspective view of core parts of the falling weight deflectometer 1 according to the invention is shown. For mobility in practical use the falling weight deflectometer 1 is mounted on a suitable carriage such as a trailer or a vehicle, known *per se* and shown schematically in figs. 6a-6f. Cf. also the various illustrations in the above-mentioned article "Faldloddets historie" incorporated herein by reference.

The falling weight deflectometer 1 comprises a drop weight 8 adapted to be dropped onto and impacting a loading transfer plate 6, from which the impact is transferred to a test surface 30 (shown in figs. 6a to 6f only), such as a pavement of e.g. Portland cement concrete or asphalt concrete, via force transmission means comprising *inter alia* a support column 5, and a load plate 2 adapted to engage the test surface 30.

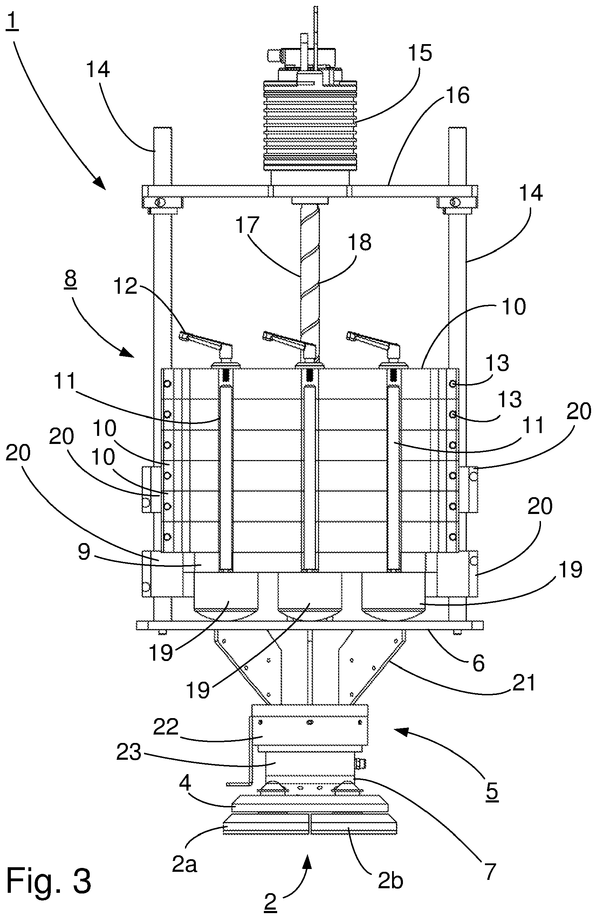

The load plate 2 is preferably circular and may, as can be seen from fig. 1, be segmented. In the preferred embodiment there are four segments of which three segments 2a, 2b, 2c are visible. Evidently, any suitable number of segments including one, i.e. a single circular load plate 2, may be used. As best seen in fig. 3, the segments 2a, 2b, 2c of the load plate 2 are connected via spacers 3 to an intermediate plate 4, allowing each of the segments 2a, 2b, 2c to have a small degree of freedom in order to allow the overall load plate 2 to accomodate irregularities the test surface 30, even if the test surface 30 is not entirely plane or horizontal. The intermediate plate 4 preferably also has some degree of freedom with respect to the support column 5 in order to adapt to inclinations in the test surface 30.

The intermediate plate 4 is connected to loading transfer plate 6 via further support means 21, 22, 23 forming part of the support column 5. The further support means also includes a force transducer 7, such as a load cell, adapted to measure the forces in the impact of the falling drop weight 8 onto the loading transfer plate 6.

The drop weight 8 comprises a base plate 9 onto which a number of individual removable weights 10 may be placed in order to achieve a desired amount of weight for a test to be performed. The weight of the each of the removable weights 10 are preferably adapted to manual handling, and the drop weight 8 may thus comprise a substantial number of removable weights 10 stacked onto each other. In order to secure the stacks of removable weights 10 on top of the base plate 9, securing means 11, 12 are also provided. For the manual handling the removable weights 10 are preferably provided with carrier handles 13.

Below the base plate 9 a number of elastic buffers 19 are provided in order to cushion the impact and obtain a suitable sinusoidal half-wave in the impact. The elastic buffers 19 are preferably interchangeable, allowing their number, their size, elastic properties etc. to be varied in accordance with a given desired amount of weight of the drop weight 8, and pulse width of the sinusoidal half-wave.

At two sides of the base plate 9 linear bearings 20, e.g. comprising ball bearings, are provided in order to allow smooth sliding motion of the base plate 9 carrying the weights 10 along two vertical columns 14 secured to the loading transfer plate 6, thus guiding the up and down motions of the drop weight 8 when it is dropped or lifted.

As for the lifting, the present invention employs an electric motor 15, mounted on a horizontal cross-beam 16 at the upper end of the two vertical columns 14. The electric motor 15 is preferably connected directly to a threaded spindle 17. The threaded spindle 17, in turn, is in a permanent engagement with a corresponding means in the base plate 9. This corresponding means is adapted to provide low friction, and may e.g. be a leadscrew, roller screw, or ball screw engagement. Low friction is important as the engagement between the threaded spindle 17 and the base plate 9 should not be self-locking. For the same reason the lead of the thread 18 of the spindle 17 is high, e.g. having lead angle exceeding 40°, preferably exceeding 50°.

Having such a high lead and low friction allows the drop weight 8 to be dropped in almost free fall. Evidently, there will be friction and some braking effect from having to turn the electric motor 15, but this may either be neglected or compensated for by supplying the electric motor 15 with sufficient current to counteract the braking effect.

Conversely, the lead of the thread 18 of the threaded spindle 17 should not be so high that the maximum torque that the electric motor 15 may provide is insufficient to lift the drop weight 8.

The preferred motor is preferably a permanent magnet torque motor such as an ETEL TMB Torque Motor, available from ETEL S.A., Zone Industrielle, CH - 2112 Môtiers, Switzerland. Such motors allow very high torque which, in turn, allows the use of a direct drive of a spindle with a very high lead. Moreover, such motors provide excellent positional control over the spindle 17, in turn allowing the impact to be controlled very accurately, e.g. when using a robotic controller, robotic controllers typically providing both a torque control mode and a position control mode. Evidently, an electric motor 15 providing lower torque could be used in connection with a gear mechanism, but in order to keep the movable parts to a minimum and the mechanism simple, a direct drive is preferred.

With this drive arrangement it becomes much faster to perform the measurements, because consecutive impacts at the same location may be made in more rapid succession.

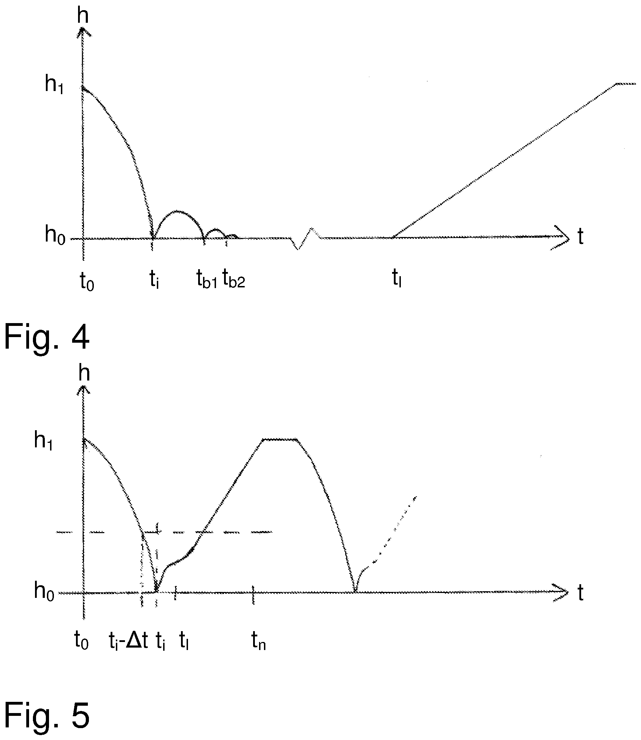

Turning now to fig. 4, a diagram of a drop sequence with the prior art falling weight deflectometer is shown. For easy comparison with fig. 5 later the sequence starts with the drop weight lifted to and held in a predetermined height h₁ from which the drop weight is to be dropped, e.g. 40 cm for a 750 kg drop weight. When the drop weight is released it will fall in virtually free fall, impact the loading transfer plate, and compress the elastic buffers at height h₀ at time of impact tᵢ. Due to the elastic properties of mainly the buffers the drop weight will bounce back and repeatedly impact the loading transfer plate at times t_{b1}, t_{b2} etc. until it comes to rest on the load transfer plate. Having come to rest the drop weight is manually attached to the lifting means again, and at tₗ the hydraulic lifting process may start again. It should be noted that fig. 4 is schematic and that the duration between t_{b2} and tₗ is in fact several seconds as to compared to the approximately 0,285 seconds involved in the free fall from 40 cm.

In fig. 5 a drop using the present invention is illustrated in a diagram similar to that of fig. 4. The drop weight 8 is dropped from the predetermined height h₁, falls in a virtually free fall, and impacts the loading transfer plate 6, at tᵢ. Since the free fall corresponds to that of the prior art FWDs, the fall is represented by similar parabolas in the time vs. elevation diagrams of fig. 4 and fig. 5. As compared to the prior, the free fall is, however, not entirely free because base plate 9 of the drop weight 8 is in permanent engagement with the threaded spindle 17. When dropped, the drop weight 8 will therefore inevitably rotate the electric motor 15, which even with open electrical supply terminals will tend to brake the fall, i.e. resist the spindle rotation caused by the falling drop weight 8. This can be compensated for by supplying the motor with sufficient current to turn the motor 15 and the threaded spindle 17 in accordance with the free fall. This is quite easily done due to the ability to accurately control the motor positioning or the torque. The theoretical position of the drop weight 8 through a free fall is easily calculated, and converted to corresponding positions of the threaded spindle 17, and, in turn, the motor 15. In order to achieve a truly free fall it would evidently also be possible to incorporate a clutch mechanism between the drop weight 8 and the motor 15, but for the lifting purposes it is preferred to have a permanent engagement. Alternatively the motor could be set to provide zero torque.

However, in order to avoid any influence of the motor controller on the impact, the motor controller is preferably disabled at a predetermined height h₂ before impact, i.e. at tᵢ-Δt, so that the motor basically has open supply terminals at impact. This short time Δt, where the braking is not counteracted can be considered negligible for the impact. In any case the actual force of the impact is measured using the force transducer 7.

Upon impact the drop weight 8 bounces back due to elastic properties *inter alia* in the buffers 19. Since the threaded spindle 17 is in permanent engagement with the base plate 9 of the drop weight 8, it is not necessary to wait until the drop weight 8 settles on the loading transfer plate 6 in order to reattach a lifting means. Instead, all it takes to lift the drop weight 8 back to the drop height h₁ is to start the supply of the electric motor 15 at a suitable lift time tₗ, e.g. during the first bounce back of the drop weight 8. The duration of the lifting itself is very short, and within a few seconds at the time tₙ the drop weight 8 is back at height h1 for the next drop. This is long before the next drop would be possible with the prior art hydraulic lifting mechanism.

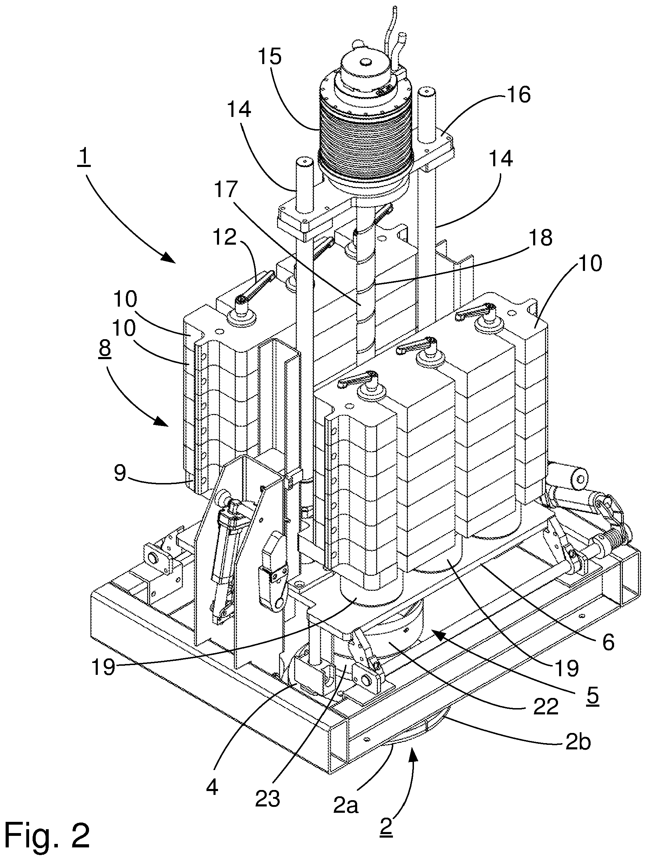

In fig. 2 a falling weight deflectometer according to the invention is mounted on a frame 24. Though not shown in fig. 2, the frame may be mounted on or form an integral part of a carriage with wheels 25 as schematically illustrated in figs. 6a-6f, such as a trailer or a self-propelled vehicle. On the carriage 24, first securing means 26 and second securing means 27 are provided. The first securing means 26 comprises an arm which may at least partially be swung below the loading transfer plate 6, allowing the loading transfer plate 6 to be secured thereon, and thus on the frame, by its own weight. The swinging motion is preferably performed by an electric actuator 28 acting on a common shaft 29 on which the arms are mounted. As can be seen from fig. 2 the first securing means 26 preferably comprises two shafts 28 each common to a pair of arms, so as to support the loading transfer plate 6 at four corners. The actuators of course need not be electrical but could be hydraulic or driven by other suitable means. Instead of simply supporting the weight of the loading transfer plate 8, the securing means 26 could of course also engage the loading plate 6 in a locking manner.

The second securing means 27 is in a similar manner adapted to be swung below the base plate 9 of the drop weight 8. Also here, one or more arms are swung by means of an electric actuator turning a shaft to which the arms are secured. Preferably also for the second securing means a double system is provided, so as engage and secure either side of the drop weight as schematically shown in figs. 6a to 6c. Likewise, as indicated in figs. 6a to 6c, the second securing means 27 may engage the drop weight 8 in a locking manner rather than just supporting the weight thereof.

As will now be explained, the use of the second securing means 27 allows the use of the electric motor 15 and the threaded spindle 17 for quickly moving the support column 5 with the load plate 2 into engagement with the test surface 30, and upon completion of a series of measurement at the same location quickly moving the support column 5 back into a secured storage position for transport to the next test location. Typically the transport distance to the next location is very short, and any time saved on transport between locations will substantially benefit the overall time necessary for testing af given stretch of pavement.

Turning now to fig. 6a the falling weight deflectometer 1 is shown in a situation with the drop weight 8 secured by the second securing means 27. The column 5 lifted free of the test surface 30 and the loading plate is in engagement the buffers 19 of the drop weight 8. If the electric motor 15 is suitably energized it can hold the spindle in a fixed position and thus carry the weight of the loading plate 6 and the support column 5. In this situation it is possible to disengage the first securing means 26. Upon disengagement of the first securing means 26 but with the drop weight 8 still secured the support column 5 forms a travelling nut arrangement with the spindle 17, and may thus be moved up and down by rotation of the spindle. Simply by energizing the electric motor 15 suitably, the support column 5 may be lowered into engagement with the test surface 30 as illustrated in figs. 6b and 6c. Once engaging the test surface 30 the support column 5 rests firmly thereon. In this situation, as illustrated in fig. 6d, the second securing means 25 may be disengaged from the drop weight, the drop weight 8 now being held by the suitably energized electric motor 15. Currently a robotic controller is preferred. Such a robotic controller provides both a position mode and a torque mode for the control of the motor. During lifting and holding the drop weight 8, the position mode would be used, whereas the torque mode would be used during the drop sequence.

From the situation of fig. 6d the drop weight 8 may be lifted to the drop position shown in fig. 6e, i.e. the height h₁ as explained in conjunction with fig 5, by the electric motor 15 turning the spindle 17. Upon that the drop is performed and the drop weight impacts on the loading plate 6 as illustrated in fig. 6f and as explained in conjunction with fig. 5 above. When a suitable number of drops have been made at one location, the process may be reversed, i.e. the drop weight 8 positioned at the height illustrated in fig. 6d where it can be secured as illustrated in fig. 6c. Again acting as a travelling nut the support column may be lifted from the test surface 30 and secured in a transport position. All of the above may be controlled electrically and therefore renders itself to automation, without other manual intervention than e.g. the pushing of a few buttons. Both the lowering and the lifting of the support column 5 may be performed very quickly, e.g. in the range of 1½ to 2 seconds, which is a substantial improvement over the prior art setup time.Apart from the possibility of making impacts in rapid succession, the present invention provides additional benefits. Because a torque motor with suitable control has a very good position in addition to the high torque it can produce, it can provide much more than the lifting action. Basically, it can move the drop weight 8 to any desired position between h₀ and h₁ through controlled acceleration and deceleration and hold it at that desired position. This means that unlike the prior art falling weight deflectometers the falling weight deflectometer according to the present invention is in fact not restricted to free falls. Instead of free fall a higher or lower downward acceleration than the acceleration in free fall may be provided.

One use for this could be shaping the impact, if rather than disabling the motor controller just before impact, as suggested above, the motor controller maintains engaged, the good position control allows the motor controller to control the force during an impact. Impacts with the desired half-sinusoidal impact force characteristics but long durations, e.g. up to 10 seconds may thus be achieved. Another use could be removing the load of the carriage of the FWD on the ground in impact. If in the last instances before impact the falling drop weight 8 is accelerated beyond the free fall acceleration, inertia will lift the carriage off the ground, thus allowing influences from the weight of the carriage on the measurements to be avoided.

## Claims

1. A falling weight deflectometer (1) comprising
a load plate (2) adapted to engage a test surface (30),
a force transmission means (5) adapted to transmit a force to the load plate (2),
a buffer means (19),
a drop weight (8) adapted to impact said force transmission means (5) via said buffer means (19) so as to provide a force to be transmitted to said load plate (2) via said force transmission means (5),
a guide means (14) for guiding said drop weight (8) towards said force transmission means (5), and a lifting means for lifting said drop weight to a predetermined height above said force transmission means (5),
**characterized in that**
said lifting means comprises an electric motor (15) driving a threaded spindle (17) in engagement with the drop weight (8), and **in that**
the threaded spindle (17) is in a non self-locking engagement with the drop weight (8).

2. A falling weight deflectometer (1) according to claim 1, wherein the threaded spindle (17) is in permanent engagement with the drop weight (8).

3. A falling weight deflectometer (1) according to any one of the preceding claims, wherein the threaded spindle (17) is in permanent engagement with the electric motor (15).

4. A falling weight deflectometer (1) according to claim 3, wherein the threaded spindle (17) is directly driven by the electric motor (15).

5. A falling weight deflectometer (1) according to any one of the preceding claims, wherein the engagement between the threaded spindle (17) and the drop weight (8) is selected among leadscrew, roller screw, or ball screw engagement.

6. A falling weight deflectometer (1) according to any one of claims 1 or 5, wherein the threaded spindle (17) has a lead angle exceeding 40°, preferably exceeding 50°.

7. A falling weight deflectometer (1) according to any one of the preceding claims further comprising a motor controller for the electric motor (15), said motor controller being adapted to control the speed of the falling drop weight (8).

8. A falling weight deflectometer (1) according to any one of claims 1 to 7, further comprising a motor controller for the electric motor (15), said motor controller (15) being adapted to control the position of the falling drop weight (8).

9. A falling weight deflectometer (1) according to any one of the preceding claims, further comprising a frame (24) and means (26, 27) for securing the drop weight (8) with respect to the frame (24).

## Patentansprüche

1. Fallgewichts-Deflektometer (FWD, Falling Weight Deflectometer) (1), umfassend
eine Lastplatte (2), die ausgelegt ist, eine Prüffläche (30) zu kontaktieren,
ein Kraftübertragungsmittel (5), das ausgelegt ist, eine Kraft auf die Lastplatte (2) zu übertragen,
ein Puffermittel (19),
ein Fallgewicht (8), das ausgelegt ist, auf das Kraftübertragungsmittel (5) über das Puffermittel (19) aufzuprallen, um eine Kraft aufzubringen, die über das Kraftübertragungsmittel (5) auf die Lastplatte (2) zu übertragen ist,
ein Führungsmittel (14) zum Führen des Fallgewichts (8) hin zu dem Kraftübertragungsmittel (5), und ein Hebemittel zum Anheben des Fallgewichts auf eine vorbestimmte Höhe über dem Kraftübertragungsmittel (5),
**dadurch gekennzeichnet, dass**
das Hebemittel einen Elektromotor (15) umfasst, der eine Gewindespindel (17) antreibt, die mit dem Fallgewicht (8) in Eingriff ist, und dass
die Gewindespindel (17) in einem nicht automatisch einrastenden Eingriff mit dem Fallgewicht (8) ist.

2. Fallgewichts-Deflektometer (1) nach Anspruch 1, wobei die Gewindespindel (17) in einem dauerhaften Eingriff mit dem Fallgewicht (8) ist.

3. Fallgewichts-Deflektometer (1) nach einem der vorhergehenden Ansprüche, wobei die Gewindespindel (17) in einem dauerhaften Eingriff mit dem Elektromotor (15) ist.

4. Fallgewichts-Deflektometer (1) nach Anspruch 3, wobei die Gewindespindel (17) direkt durch den Elektromotor (15) angetrieben ist.

5. Fallgewichts-Deflektometer (1) nach einem der vorhergehenden Ansprüche, wobei der Eingriff zwischen der Gewindespindel (17) und dem Fallgewicht (8) aus einem Eingriff eines Führungsgewindes, eines Rollengewindes oder eines Kugelgewindes ausgewählt ist.

6. Fallgewichts-Deflektometer (1) nach einem der Ansprüche 1 oder 5, wobei die Gewindespindel (17) einen Steigungswinkel aufweist, der 40° übersteigt, vorzugsweise 50° übersteigt.

7. Fallgewichts-Deflektometer (1) nach einem der vorhergehenden Ansprüche, ferner umfassend eine Motorsteuerung für den Elektromotor (15), wobei die Motorsteuerung ausgelegt ist, die Geschwindigkeit des fallenden Fallgewichts (8) zu steuern.

8. Fallgewichts-Deflektometer (1) nach einem der Ansprüche 1 bis 7, ferner umfassend eine Motorsteuerung für den Elektromotor (15), wobei die Motorsteuerung (15) ausgelegt ist, die Position des fallenden Fallgewichts (8) zu steuern.

9. Fallgewichts-Deflektometer (1) nach einem der vorhergehenden Ansprüche, ferner umfassend einen Rahmen (24) und Mittel (26, 27) zum Sichern des Fallgewichts (8) in Bezug auf den Rahmen (24).

## Revendications

1. Déflectomètre à masse tombante (1) comprenant
un plateau de charge (2) agencé pour venir en contact avec une surface d'essai (30),
un moyen de transmission de force (5) agencé pour transmettre une force au plateau de charge (2),
un moyen formant tampon (19),
une masse tombante (8) agencée pour heurter ledit moyen de transmission de force (5) par le biais dudit moyen formant tampon (19) de manière à produire une force devant être transmise audit plateau de charge (2) à l'aide dudit moyen de transmission de force (5),
un moyen de guidage (14) pour guider ladite masse tombante (8) vers ledit moyen de transmission de force (5), et un moyen de levage pour soulever ladite masse tombante jusqu'à une hauteur prédéterminée au-dessus dudit moyen de transmission de force (5),
**caractérisé en ce que**
ledit moyen de levage comprend un moteur électrique (15) entraînant une broche filetée (17) en prise avec la masse tombante (8), et **en ce que**
la broche filetée (17) est en prise non autobloquante avec la masse tombante (8).

2. Déflectomètre à masse tombante (1) selon la revendication 1, dans lequel la broche filetée (17) est en prise permanente avec la masse tombante (8).

3. Déflectomètre à masse tombante (1) selon l'une quelconque des revendications précédentes, dans lequel la broche filetée (17) est en prise permanente avec le moteur électrique (15).

4. Déflectomètre à masse tombante (1) selon la revendication 3, dans lequel la broche filetée (17) est actionnée directement par le moteur électrique (15).

5. Déflectomètre à masse tombante (1) selon l'une quelconque des revendications précédentes, dans lequel la prise entre la tige filetée (17) et la masse tombante (8) est choisie parmi une prise par vis sans tête, vis à rouleaux ou vis à billes.

6. Déflectomètre à masse tombante (1) selon l'une quelconque des revendications 1 ou 5, dans lequel la broche filetée (17) a un angle d'attaque supérieur à 40 °, de préférence supérieur à 50 °.

7. Déflectomètre à masse tombante (1) selon l'une quelconque des revendications précédentes, comprenant en outre un dispositif de contrôle de moteur pour le moteur électrique (15), ledit dispositif de contrôle de moteur étant agencé pour contrôler la vitesse de la masse tombante (8).

8. Déflectomètre à masse tombante (1) selon l'une quelconque des revendications 1 à 7, comprenant en outre un dispositif de contrôle de moteur pour le moteur électrique (15), ledit dispositif de contrôle de moteur étant agencé pour contrôler la position de la masse tombante (8).

9. Déflectomètre à masse tombante (1) selon l'une quelconque des revendications précédentes, comprenant en outre un châssis (24) et des moyens (26, 27) pour sécuriser la masse tombante (8) par rapport au châssis (24) .
